# EUROPEAN PATENT SPECIFICATION

(11) **EP 0 825 860 B1**
(45) Date of publication and mention of the grant of the patent: **26.09.2001**
(21) Application number: 96919679.9
(22) Date of filing: 02.05.1996
(51) Int. Cl.: A61K 31/43, A61K 31/424

(54) **USE OF A COMPOSITION COMPRISING AMOXYCILLIN AND CLAVULANIC ACID FOR THE MANUFACTURE OF A MEDICAMENT FOR THE TREATMENT OF BACTERIAL INFECTIONS IN PAEDIATRIC PATIENTS**
VERWENDUNG EINER ZUBEREITUNG ENTHALTEND AMOXYCILLIN UND CLAVULANSÄURE ZUR HERSTELLUNG EINES MEDIKAMENTS ZUR BEHANDLUNG VON BAKTERIELLEN INFEKTIONEN BEI PÄDIATRISCHEN PATIENTEN
UTILISATION D'UNE COMPOSITION A BASE D'AMOXYCILLINE ET D'ACIDE CLAVULANIQUE POUR LA PREPARATION D'UN MEDICAMENTE POUR LE TRAITEMENT DES INFECTIONS BACTERIELLES POUR ENFANTS

(30) Priority: 03.05.1995 GB 9508989; 18.11.1995 GB 9523655
(43) Date of publication of application: 04.03.1998
(60) Divisional application: 00200560.1
(73) Proprietor: SMITHKLINE BEECHAM PLC, Brentford, Middlesex TW8 9EP (GB); SMITHKLINE BEECHAM CORPORATION, Philadelphia Pennsylvania 19103 (US)
(72) Inventor: BAX, Richard, Peregrine, SmithKline Beecham Pharma, Harlow, Essex CM19 5AW (GB); RAMSEY, Mike, Gale, SmithKline Beecham Pharma., Bristol, TN 37620 (US)
(74) Representative: Connell, Anthony Christopher
(86) International application number: EP9601881
(87) International publication number: WO9634605

(56) References cited:
- EP-A- 0 080 862
- WO-A-91/15197
- WO-A-95/28927
- WO-A-96/04907
- GB-A- 2 005 538
- US-A- 4 537 887
- J.INT.MED.RES., vol. 17, no. 2, 1989, pages 168-171, XP002010124 RUBERTO,U., ET AL.: "Amoxycillin and clavulanic acid in the treatment of urinary tract infections in children"
- CAN.MED.ASSOC.J., vol. 142, no. 2, 15 January 1990, pages 115-118, XP002010125 FELDMANN,W.,ET AL.: "Twice-daily antibiotics in the treatment of acute otitis media: trimethoprim-sulfamethoxazole versus amoxicillin-clavulanate"
- EUR.J.CLIN.MICROBIOL.INFECT.DIS., vol. 12, no. 5, May 1993, pages 319-324, XP002010126 JACOBSON,S., ET AL.: "Evaluation of amoxicillin clavulanate twice daily versus thrice daily in the treatment of otitis media in children"

## Description

This invention relates to the use of a combination of the antibiotic amoxycillin and the beta-lactamase inhibitor potassium clavulanate in treating paediatric bacterial infections.

The combination of the antibiotic amoxycillin, as amoxycillin trihydrate, and potassium clavulanate, is a well known and widely used oral medicament for bacterial infections, marketed by SmithKline Beecham in many countries under the trade mark Augmentin.

Regulatory approval has been obtained, for instance in the UK and US, for tablets containing amoxycillin (250mg) and potassium clavulanate (125mg) (ratio 2:1), in a three times daily dosing schedule (tid), so that the daily dose of amoxycillin and potassium clavulanate is 750mg and 375mg respectively, the weights being expressed as the free parent acids amoxycillin and clavulanic acid, this manner of expression being used throughout. In severe infections, the ratio is changed to 4:1, so that the daily doses of amoxycillin and potassium clavulanate are 1500mg and 375mg respectively. In other countries such as Italy and Spain, tablets containing amoxycillin (875mg) and potassium clavulanate (125mg) (ratio 7:1) are approved for twice daily dosing (bid). In France, sachets comprising amoxycillin (1000mg) and potassium clavulanate (125mg) (ratio 8:1) are marketed, for reconstitution with water prior to use, as an individual dosage for adults.

For children, it is preferred to provide the combination as a powder which is reconstituted prior to use as a liquid aqueous suspension. The usual recommended daily dosage is 20/5mg/kg/day (UK and USA) or 30/7.5mg/kg/day (Continental Europe) amoxycillin/potassium clavulanate (ratio 4:1), in divided doses every eight hours. For more severe infections such as otitis media, sinusitis and lower respiraotory tract infections, the recommended dose is 40/10mg/kg/day (UK and USA) or 60/15mg/kg/day (Continental Europe), in three divided doses. For convenience, the powders are provided in amounts which are made up into a range of volumes of suspension so that a small volume, typically about 5ml, contains a unit dosage. In other countries such as Italy, approval has also been given for using the higher strength paediatric formulation on a twice a day (bid) dosing schedule.

Although it is recognised to be more convenient to be able to recommend a bid dosing schedule for paediatric formulations, to avoid having to give medicine during the middle of the day when the child may be at school, not all drug substances have pharmocokinetics which are compatible with such a regimen.

In addition, gastric intolerance, manifested in symptons such as loose stools, is perceived in some countries to be a side effect associated with the use of amoxycillin/potassium clavulanate in the present tid dosage regimes. Accordingly, any measures, such as revised dosage regimes, which can mitigate this would be advantageous.

There were a limited number of reports of studies using the existing 4:1 amoxycillin/clavulante suspension formulation in a BID regimen, rather than the approved TID regimen, with mixed results for efficacy and a trend towards increased adverse events. Thus, Jacobsson et al (Eur J clin Microbiol Infect Dis, 1993, 12(5), 319-324) describe a clinical trial using the existing 4:1 amoxycillin/clavulante suspension formulation in a BID regimen, for treating acute otitis media in children, at an average dosage of about 30 mg/kg/day. They report comparable efficacy to the TID regimen, with a trend towards an increased rate of diarrhoea. In a further study on acute otitis media, Feldman et al (Can Med Assocn J, 1990, 142(2), 115-118) compared the existing 4:1 amoxycillin/clavulante suspension formulation in a BID regimen (at a dosage of 40mg/kg/day amoxycillin) with a trimethoprin-sulfamethoxazole BID regimen and found it to be less effective. Ruberto at al (I Ijnt Med Res, 1989, 17(2), 168-171) describe a clinical study of the treatment of urinary tract infections in children using the existing 4:1 amoxycillin/clavulante suspension formulation in a BID regimen. The infections were cleared in 89% of the cases. In five, gastro-intestinal disorders were regressed by dosing at 8h rather than 12h intervals.

The present invention therefore provides for the use of amoxycillin trihydrate and potassium clavulanate in combination, in a weight ratio of 7:1 the weights being expressed as the free parent acids amoxycillin and clavulanic acid, for the manufacture of a paediatric medicament for treating bacterial infections in paediatric patients which medicament is administered twice daily (bid), at a dosage of between 20 and 70 mg/kg/day of amoxycillin and *a pro rata* amount of clavulanic acid., suitably 40 and 70mg/kg/day, of amoxycillin and *pro rata* amounts of clavulanic acid.

The term 'paediatric' as used herein means children in the age range from 0 to about 12 years.

The use is suitable for all infections for which the combination of amoxycillin trihydrate and potassium clavulanate is normally prescribed, for instance infections of the upper and lower respiratory tract, urinary tract, skin and skin structures, for example *otitis media.*

Suitabably the twice daily (bid) administration is at 12 hour intervals, although a greater or lesser interval between administrations may be used.

Representative daily amounts of amoxycillin/potassium clavulanate include 25/3.6, 35/5, 45/6.4 and 70/10±10%mg/kg/day.

Pharmaceutical formulation suitable for use in the present invention comprise amoxycillin trihydrate and potassium clavulanate in combination, in a weight ratio of 7:1, the weights being expressed as the free parent acids amoxycillin and clavulanic acid, and in quantities such as to provide between between 20 and 70mg/kg/day, suitably 40 and 70mg/kg/day, of amoxycillin and *pro rata* amounts of clavulanic acid.

Such formulations are preferably provided in the form of a dry powder or granule formulation for reconstitution into an aqueous suspension with water or other suitable aqueous media, shortly before administration. The present invention covers such dry powder and granule formulations as well as liquid aqueous preparations.

Such dry formulations may be provided in a substantially air-tight container such as a bottle or sachet, and this container may suitably include a desiccant to protect the potassium clavulanate from degradation by atmospheric water vapour. Potassium clavulanate is highly moisture sensitive, and the formulations of this invention should be made under conditions of relative humidty (RH) as low as possible, preferably 30% RH or less.

Such formulations are provided for bid administration, which ideally may comprise two administrations at 12 hour dosage intervals, although a greater or lesser interval between administrations may be used.

Suitably, such formulations are provided in amounts such that the liquid aqueous suspension contains in a convenient volume, typically within the range 2 to 10ml, preferably about 5ml, of suspension, a unit dosage of amoxycillin and potassium clavulanate. The volume may be measured by any conventional measuring device such as a spoon, syringe or graduated measuring cup. Unit dosages typically lie within the range 50 to 800mg of amoxycillin plus *pro rata* amounts of potassium clavulanate. It will be appreciated that the appropriate unit dosage will be at the discretion of the physician and will depend *inter alia* upon the age and weight of the patient and the nature and severity of the infection to be treated.

It is found to be convenient to provide paediatric formulations in a lower (for mild-moderate infections) and a higher strength (for severe infections). The suitable formulations, when made up as aqueous liquid suspensions, will contain from 100 to 400mg, or 200 to 800mg amoxycillin per 5ml of suspension plus *pro rata* amounts of potassium clavulanate. Representative examples include:

| | |
|---|---|
| amoxycillin | potassium clavulanate |
| 200 | 28.5/5m1 suspension |
| 400 | 57; |

within a tolerance of ±10%.

In a representative example, for a unit dosages of 5ml, the suspension is made up to a total volume of 100ml. It will however be appreciated that a range of total volumes may be used, to adjust the amount in a unit dose to an amount appropriate for the individual patient. It will be appreciated that it may also be convenient to provide a higher strength formulation to a patient with a larger body weighty so that the unit volume is kept reasonable.

The formulation will normally comprise, in addition to its active materials amoxycillin trihydrate and potassium clavulanate, excipients which are standard in the field of paediatric pharmaceutical oral suspensions. These will be used in generally standard proportions, and at generally standard particle sizes and grades etc.. Such excipients may comprise suspending aids, glidants (to aid filling), diluents, bulking agent, flavours, sweeteners, stabilisers, and, in the case of dry formulations for make-up to an aqueous suspension, an edible desiccant to assist preservation of the potassium clavulanate against hydrolysis by atmospheric moisture on storage. Potassium clavulanate is normally supplied in admixture with silicon dioxide as diluent. Suitable excipients for use include xanthan gum (suspension aid), colloidal silica (glidant), succinic acid (stabiliser), aspartame (sweetener), hydroxypropylmethylcellulose (suspension aid) and silicon dioxide (desiccant, diluent for potassium clavulanate and bulking agent. Flavours may comprise common flavours such as orange, banana, raspberry and golden syrup, or mixtures thereof, to suit local requirements.

Mannitol is often used in pharmaceutical formulations as an excipient. It is however recognised to have, at least at certain levels, a diuretic effect. It has found to be advantageous to avoid the use of excessive amounts of mannitol in formulations comprising amoxycillin/potassium clavulanate, as it is thought that this may be associated with reduced levels of gastric irritancy.

Generally the proportion of active materials amoxycillin trihydrate and potassium clavulanate in a dry formulation for make-up with aqueous media into a suspension formulation may be around 35-60, e.g. 35-50 wt%.

The formulation may be manufactured using techniques which are generally conventional in the field of manufacture of paediatric suspension formulations and manufacture of dry formulations for reconstitution into such suspensions. For example a suitable technique is that of mixing dry powdered or granulated ingredients for loading into a suitable container.

### Reference Example 1

Two formulations of this invention having a composition as listed below were prepared using conventional techniques as dry powder mixtures. The proportions of ingredients are expressed as mg/5ml dose of reconstituted aqueous suspension:

| **Ingredient** | **mg/5ml** | **mg/5ml** |
|---|---|---|
| amoxycillin trihydrate* | 408.0 | 204.0 |
| potassium clavulanate* | 61.56 | 30.78 |
| xanthan gum | 12.5 | 12.5 |
| colloidal silica | 25.0 | 25.0 |
| succinnic acid | 0.84 | 0.84 |
| orange flavour | 26.25 | 26.25 |
| golden syrup flavour | 23.75 | 23.75 |
| aspartame | 12.50 | 12.50 |
| hydroxypropylmethylcellulose | 79.65 | 79.65 |
| silicon dioxide | to 885.5 | to 537.5 |

| | | |
|---|---|---|
| * expressed as free acid equivalent. | | |

The above two formulations were manufactured in 100 kg batches.

### Clinical Trial - A

In a multicentre randomized trial, the safety and efficacy in the treatment of acute Otitis Media in children of formulations according to the present invention and comprising amoxycillin/potassium clavulanate in a ratio of 7:1, at a level of 45/6.4 mg/kg/day (ratio 7:1) and in divided doses q12h (BID) were compared with a currently approved US formulation comprising amoxycillin/potassium clavulanate in a ratio 4:1 administered at a level of 40/10mg/kg/day amoxycillin/potassium clavulanate acid in divided doses q 8h (TID). 287 children received the BID regimen for 10 days and 288 children received the TID regimen for 10 days. The formulations according to the present invention were mannitol-free whilst the currently approved US formulation contained mannitol.

The BID regimen was shown to be as safe as the TID regimen. Standard diary cards were used to assess the incidence of incidence of protocol-defined diarrhoea (i.e. 3 or more watery stools in one day, or 2 watery stools per day for two consecutive days. This was found to be significantly lower for the BID regimen (7.9%) when compared with the TID regimen (22.2%) [95% CI: (-20.5%, -8.1%)]. Similar trends were observed for withdrawals due to diarrhoea (2.8% and 7.6% respectively; p = 0.009), confirming improved patient tolerance compared with the current regimen.

The per protocol clinical success rates at the end of therapy (days 12 to 14) were equivalent for the BID regimen (86.5%) and the TID regimen (78.88%). Similar trends in efficacy were noted at follow-up (days 32 to 38).

### Formulations used

| **Ingredient** | **bid mg/5ml** | **bid mg/5ml** | **tid mg/5ml** | **tid mg/5ml** |
|---|---|---|---|---|
| amoxycillin trihydrate* | 408.0 | 204.0 | 130.00 | 260.00 |
| potassium clavulanate* | 61.56 | 30.78 | 35.00 | 70.00 |
| xanthan gum | 12.5 | 12.5 | 15.00 | 15.00 |
| sodium saccharin | | | 4.00 | 4.00 |
| colloidal silica | 25.0 | 25.0 | 25.00 | 25.00 |
| succinnic acid | 0.84 | 0.84 | 0.85 | 0.85 |
| banana flavour | | | 20.00 | |
| orange flavour | 26.25 | 26.25 | | 23.00 |
| golden syrup flavour | 23.75 | 23.75 | | |
| aspartame | 12.50 | 12.50 | | |
| hydroxypropylmethylcellulose | 79.65 | 79.65 | | |
| silicon dioxide | to 885.5 | to 537.5 | 82.30 | 39.70 |
| mannitol | | | to 900.00 | to 1200.00 |

| | | | | |
|---|---|---|---|---|
| * expressed as free acid equivalent. | | | | |

### Clinical trial - B

In a multicentre randomized trial, the safety and efficacy in the treatment of acute Otitis Media in children of formulations according to the present invention and comprising amoxycillin/potassium clavulanate in a ratio of 7:1, at a level of 70/10mg/kg/day (ratio 7:1) and in two divided doses (BID) were compared with a currently approved European formulation comprising amoxycillin/potassium clavulanate in a ratio 4:1 administered at a level of 60/15mg/kg/day amoxycillin/potassium clavulanate acid in three divided doses (TID). Children aged from 2-12 years were randomised to 10 days treatment, with 231 children receiving the BID regimen and 232 children receiving the TID regimen.

The BID regimen was shown to be as safe as the TID regimen. Standard diary cards were used to assess the incidence of protocol-defined diarrhoea (i.e. 3 or more watery stools in one day, or 2 watery stools per day for two consecutive days). The overall incidence was found to be low with a lower incidence in the BID group (6.7%) than in the TID group (10.3%) although this was not statistically significant [difference - 3.6%; 95% CI (-8.72%, 1.58%)].

Clinical success rates at the end of therapy were 91.8% for the BID regimen and 90.5% for the TID regimen [difference 1.3%,: 95% CI (-3.92%, 6.43%)] and at follow-up were 80.1% for the BID group and 77.6% for the TID group [difference 2.5%; 95% CI (-4.94%, 9.94%)].

More patients in the BID group (81.3%) than in the TID group (72.8%) had at least 80% compliance over a 7-10 day treatment period [difference 10.3%; 95% CI (2.78%, 17.76%)].

### Formulations used

| **Ingredient** | **bid formulation mg/5ml** | **tid formulation mg/5ml** |
|---|---|---|
| amoxycillin trihydrate* | 400.0 | 250.00 |
| potassium clavulanate* | 59.85 | 65.63 |
| xanthan gum | 12.50 | 12.50 |
| colloidal silica | 25.00 | 25.00 |
| succinnic acid | 0.84 | 0.84 |
| orange flavour | 26.25 | 26.25 |
| raspberry flavour | | 22.50 |
| golden syrup flavour | 23.75 | 23.75 |
| aspartame | 12.50 | 12.50 |
| hydroxypropylmethylcellulose | 79.65 | 150.00 |
| silicon dioxide | to 885.5 | 125.00 |

| | | |
|---|---|---|
| * expressed as free acid equivalent. | | |

## Claims

1. The use of amoxycillin trihydrate and potassium clavulanate in combination, in a weight ratio of 7:1, the weights being expressed as the free parent acids amoxycillin and clavulanic acid, for the manufacture of a paediatric medicament for treating bacterial infections in paediatric patients which medicament is administered twice daily (bid), at a dosage of between 20 and 70 mg/kg/day of amoxycillin and a *pro rata* amount of clavulanic acid.

2. A use as claimed in claim 1 in which the dosage regimen is 70±10%mg/kg/day amoxycillin in combination with 10±10%mg/kg/day clavulanic acid.

3. A use as claimed in claim 1 in which the dosage regimen is 45±10%mg/kg/day amoxycillin in combination with 6.4±10%mg/kg/day clavulanic acid.

4. A use as claimed in claim 1 in which the dosage regimen is 35±10%mg/kg/day amoxycillin in combination with 5±10%mg/kg/day clavulanic acid.

5. A use as claimed in claim 1 in which the dosage regimen is 25±10%mg/kg/day amoxycillin in combination with 3.6±10%mg/kg/day clavulanic acid.

6. A use as claimed in claim 2 or 3 for treating acute otitis media.

## Patentansprüche

1. Verwendung von Amoxycillintrihydrat und Kaliumclavulanat in Kombination in einem Gewichtsverhältnis von 7:1, wobei die Gewichte ausgedrückt sind als die freien Stammsäuren Amoxycillin und Clavulansäure, für die Herstellung eines pädiatrischen Medikaments zur Behandlung von bakteriellen Infektionen in pädiatrischen Patienten, wobei das Medikament zwei Mal täglich (bid) in einer Dosierung zwischen 20 und 70 mg/kg/Tag Amoxycillin und einer pro rata-Menge an Clavulansäure verabreicht wird.

2. Verwendung nach Anspruch 1, wobei das Dosierungsschema 70 ± 10% mg/kg/Tag Amoxycillin in Kombination mit 10 ± 10% mg/kg/Tag Clavulansäure ist.

3. Verwendung nach Anspruch 1, wobei das Dosierungsschema 45 ± 10% mg/kg/Tag Amoxycillin in Kombination mit 6,4 ± 10% mg/kg/Tag Clavulansäure ist.

4. Verwendung nach Anspruch 1, wobei das Dosierungsschema 35 ± 10% mg/kg/Tag Amoxycillin in Kombination mit 5 ± 10% mg/kg/Tag Clavulansäure ist.

5. Verwendung nach Anspruch 1, wobei das Dosierungsschema 25 ± 10% mg/kg/Tag Amoxycillin in Kombination mit 3,6 ± 10% mg/kg/Tag Clavulansäure ist.

6. Verwendung nach Anspruch 2 oder 3 zur Behandlung einer akuten Mittelohrentzündung.

## Revendications

1. Utilisation de trihydrate d'amoxycilline et de clavulanate de potassium en association, en un rapport pondéral égal à 7:1, les poids étant exprimés en amoxycilline et acides clavulaniques sous forme des acides mères libres, pour la production d'un médicament pédiatrique destiné au traitement d'infections bactériennes chez des patients pédiatriques, médicament qui est administré deux fois par jour (bid), à une dose comprise dans l'intervalle de 20 à 70 mg/kg/jour d'amoxycilline et une quantité *pro rata* d'acide clavulanique.

2. Utilisation suivant la revendication 1, dans laquelle le régime posologique est constitué de 70 ± 10 % mg/kg/jour d'amoxycilline en association avec 10 ± 10 % mg/kg/jour d'acide clavulanique.

3. Utilisation suivant la revendication 1, dans laquelle le régime posologique est constitué de 45 ± 10 % mg/kg/jour d'amoxycilline en association avec 6,4 ± 10 % mg/kg/jour d'acide clavulanique.

4. Utilisation suivant la revendication 1, dans laquelle le régime posologique est constitué de 35 ± 10 % mg/kg/jour d'amoxycilline en association avec 5 ± 10 % mg/kg/jour d'acide clavulanique.

5. Utilisation suivant la revendication 1, dans laquelle le régime posologique est constitué de 25 ± 10 % mg/kg/jour d'amoxycilline en association avec 3,6 ± 10 % mg/kg/jour d'acide clavulanique.

6. Utilisation suivant la revendication 2 ou 3 pour le traitement de l'otite moyenne aiguë.
